# EUROPEAN PATENT SPECIFICATION

(11) **EP 3 049 017 B1**
(45) Date of publication and mention of the grant of the patent: **20.05.2020**
(21) Application number: 14777083.8
(22) Date of filing: 29.09.2014
(51) Int. Cl.: A61C 8/00, A61K 6/027, C03C 4/00, C03C 3/097, A61L 27/28, A61L 27/32, A61L 27/58, C04B 41/86, C03C 8/08, C03C 8/16, C04B 41/00, C04B 41/50, C04B 111/00

(54) **IMPLANTS WITH A REMOVABLE COATING**
IMPLANTATE MIT EINER ENTFERNBAREN BESCHICHTUNG
IMPLANTS DOTÉS D'UN REVÊTEMENT RETIRABLE

(30) Priority: 27.09.2013 EP 13186437
(43) Date of publication of application: 03.08.2016
(73) Proprietor: Vita Zahnfabrik H. Rauter GmbH & Co. KG, 79713 Bad Säckingen (DE)
(72) Inventor: KIRSTEN, Armin, 79713 Bad Säckingen (DE); FISCHER, Horst, 52074 Aachen (DE); FISCHER, Jens, 8805 Richterswill (CH)
(74) Representative: dompatent von Kreisler Selting Werner - Partnerschaft von Patent- und Rechtsanwälten mbB
(86) International application number: PCT/EP2014/070732
(87) International publication number: WO 2015/044401

(56) References cited:
- EP-A1- 0 377 068
- EP-A1- 0 607 017
- WO-A1-2007/144662
- WO-A2-2009/106502
- CA-C- 2 210 070
- DE-A1- 3 248 649
- DE-A1- 19 723 723
- SI-A- 9 600 242
- US-A- 4 731 394
- US-A- 5 387 558
- US-A1- 2002 157 570
- US-A1- 2009 286 666
- US-A1- 2010 003 638

## Description

The present invention relates to an implant provided with a coating, to a mixture serving to prepare the coating, to a process for preparing the implant, and to uses thereof.

Dental implants are usually provided with a microstructure on their surface in order to promote rapid ongrowth of the bone tissue. However, because of the difficult cleaning of such a microstructure, the formation of a biofilm is favored in areas that are not covered by bones after healing, which may result in an inflammatory reaction. Peri-implantitis can lead to inflammatory degradation of the bone tissue and thus to the loss of an osseo-integrated dental implant even years after implantation [1]. Therefore, an increased bacterial colonization on the implant surface should be avoided. However, modern implant surfaces often have microstructured surfaces, which are difficult to clean and therefore are colonized by bacteria.

Modern dental implants are mostly screw-shaped artificial tooth roots that may be made of metallic or ceramic materials and serve as anchors for artificial dental crowns and bridges [2]. After a corresponding pilot hole has been drilled, the implants are screwed into the jawbone to different depths depending on the anatomical situation. The surrounding soft tissue is supposed to directly enclose the implant. In order to promote osseo-integration, i.e., to create a direct bond between the implant and bone, the surfaces of today's implants are microstructured in most cases. This can be achieved by different methods, such as corundum blasting, acid etching, anodic oxidation, plasma spraying etc. [3]. Such a microstructure promotes the proliferation, migration and differentiation of bone-forming cells and thereby accelerates the anchoring of the implant in the bone [4, 5].

Documents DE19723723 and WO2009106502 disclose dental implants comprising an organic coating which facilitates osseointegration of the implant into the bone. Documents EP0377068, EP0607017, DE 3248649 and CA2210070 disclose dental implants with glass, ceramic glass or ceramic coating allowing better osseointegration of the implant into the bone. Nevertheless, a decline of the bone is frequently observed in the immediate environment of the implant, a loss in bone height of up to about 2 mm being considered as normal [6].

Peri-implantitis is a disease that occurs in about 5-10% of today's dental implant provisions and frequently leads to a loss of the implants. It is defined as an inflammatory process that adversely affects the tissues surrounding an osseo-integrated implant and results in a degradation of supporting bone tissue [1]. Impacts caused by microorganism infestation, which mainly occurs in or on roughened implant surfaces as compared to implants with polished surfaces [7], are considered the main causes of this disease. Therefore, the plaque surrounding the implant is usually removed first in a therapy. In order to prevent the renewed formation of a biofilm or plaque, the implant surface is polished in many cases, which facilitates the necessary cleaning of the implant. After the inflammatory reaction has subsided (often supported by a local or systemic antibiotic treatment), a reconstruction of the lost bone tissue around the implant is usually sought [8-10].

Although the current implant surfaces having a microstructure promote the healing of the implant into the bone, they also increase the risk of a bacterial infection when the microstructured surface is exposed. Previous strategies for achieving an improved cleanability of exposed implant surfaces, by means of mechanical removing of the micro-structured surface such as polishing the endangered areas, are very difficult to implement after the ongrowth of the implant because of the very restricted space in the oral cavity on the one hand, and in addition, they may also lead to further complications in the area around the implant from remaining abraded dust and/or polishing media.

Challenges in the preparation of partially smooth and partially microstructured implants - as possible improvements of the aforementioned drawbacks - include the individual anatomies of patients and the individual decline of the alveolar bone. It is thus practically not useful to provide an implant only with a partially micro structured surface.

Although, on the one hand, the microstructured surface is indicated for medical reasons, the described disadvantages practically mean a contraindication of the microstructured surface on the other hand.

It is an object of the invention to provide an implant that represents a way out of the mentioned dilemma. This object of the present invention is achieved by an implant according to appended claim 1. The skilled person understands that the coating is removed under physiological conditions at sites of the implant which are exposed to the oral cavity of a mamal including humans, but not at sites which hare not exposed to the oral cavity, e.g. the bone.

The implant according to the invention has a coating with a microstructure on its surface that has an osseo-inductive effect. In areas not in direct contact with bone tissue, or where bone declines over time, the coating will gradually dissolve, especially by the influence of saliva, and a smooth implant surface results, on which a forming biofilm can be easily removed. This natural process may be supported by a mechanical or chemical treatment, which can be performed, for example, by a dentist, by skilled dental staff members, or even by the patient themselves.

The microstructured implant surface is converted to a smooth surface exactly in those areas of the implant, if possible, where simple cleaning is necessary. The present invention enables the gradual conversion of a rough implant surface to a smooth implant surface by the saliva flowing around the implant in those areas where the implant protrudes from the bone. This is enabled by coating a smooth implant surface with a degradable material having a microstructured surface. Mechanically or chemically removing the coating is also conceivable. Thus, a rough implant surface is converted to a smooth surface exactly where the microstructure is undesirable in an individual manner in terms of patients and situations.

To date, coatings have been applied to dental implants mainly for promoting osseo-integration (e.g., hydroxylapatite), in which the degradation and removal of such layers was to be avoided, because the layers lost their function thereby. This general understanding is disclosed e.g. in DE 19723723 A1, WO 2009/106502 A2, US 2010/003638 A1, EP 0377068 A1, EP0607017 A1 and DE 3248649 A1. There are described various methods and compositions for coating or manufacturing implants with certain surfaces but the surfaces are designed for permanent staying on the implants, however, removability of the surfaces with a micro structure is not mentioned as well as is not desired. In contrast, in the present invention, a defined degradation of the coating, for example, by flowing saliva, is sought in order to optimize the properties of the implant. This also opens up a strategy for combating peri-implantitis. In the present invention, this disease is not treated only when it has already occurred, but it is prevented in advance by the design and the properties of the dental implant. In contrast to current therapies, the prophylactic effect is paramount. The advantages are obvious.

Despite of prefabricated parts, which is always the case with industrially fabricated implants, an solution to the described problem that is individual in terms of both patients and situations is possible. Complicated therapies for treating implant loss from peri-implantitis can be avoided by this invention, so that the manual polishing of the exposed microstructured implant surfaces by the dentist is dispensed with, and the osseo-integration of the implant is not adversely affected. In comparison with the polishing of the implant surface, there is a much lesser risk of contaminating the surrounding tissue even if the coating is detached mechanically or chemically.

According to the invention, the coating is removed chemically by contact with fluids occurring in the oral cavity.

In yet another embodiment, the implant according to the invention is made of metal, ceramic or plastic or combinations thereof.

According to the invention, the coating is a coating of inorganic material.

According to the invention, the inorganic material is selected from the group consisting of ceramic, glass ceramic, glass and metals. Said ceramic may include a calcium phosphate, a calcium carbonate, a calcium silicate, or mixtures thereof, said glass ceramic may be based on an alkali silicate glass, alkaline earth silicate glass, phosphate glass, or mixtures thereof, and may be in a partially or completely crystallized form. The glass may be an alkali silicate glass, alkaline earth silicate glass, phosphate glass, or mixtures thereof.

The coefficient of thermal expansion of the coating of the implant according to the invention is, in particular, ± 1 ppm/K, based on the coefficient of thermal expansion of the substrate material.

The implant according to the invention is typically coated with a mixture, to which the present invention also relates in terms of an intermediate product, containing or consisting of
SiO₂ in amounts of from 40 to 50% by mass;
MgO in amounts of from 25 to 30% by mass;
CaO in amounts of from 0 to 5% by mass;
K₂O in amounts of from 18 to 24% by mass;
P₂O₅ in amounts of from 0 to 10% by mass;
Na₂O in amounts of from 0 to 5% by mass;
ZrO₂ in amounts of from 0 to 5% by mass;
SrO in amounts of from 0 to 5% by mass;
BaO in amounts of from 0 to 5% by mass;
Al₂O₃ in amounts of from 0 to 3% by mass;
Y₂O₃ in amounts of from 0 to 5% by mass;
CaF₂ in amounts of from 0 to 5% by mass;
TiO₂ in amounts of from 0 to 5% by mass;
Ag in amounts of from 0 to 5% by mass.

Other elements may also be contained in traces. US 2002/157570 A1 discloses compositions having an amount of 5% F. This is substantially higher than the F amount in the mixture of the invention (present invention ca 1%). Similarly the US 5,387,558 is of no relevance.

In an alternative, the mixture may have or may consist of the following composition, in particular:
SiO₂ in amounts of from 40 to 50% by mass;
MgO in amounts of from 14 to 20% by mass;
CaO in amounts of from 20 to 25% by mass, in particular 20 to 23 % by mass;
K₂O in amounts of from 7 to 12% by mass;
P₂O₅ in amounts of from 0 to 10% by mass, in particular 1 to 10 % by mass;
Na₂O in amounts of from 0 to 5% by mass;
ZrO₂ in amounts of from 0 to 5% by mass;
SrO in amounts of from 0 to 5% by mass;
BaO in amounts of from 0 to 5% by mass;
Al₂O₃ in amounts of from 0 to 3% by mass;
Y₂O₃ in amounts of from 0 to 5% by mass;
CaF₂ in amounts of from 0 to 5% by mass;
TiO₂ in amounts of from 0 to 5% by mass;
Ag in amounts of from 0 to 5% by mass.

Other elements may also be contained in traces. This alternative has the same main component, silicon dioxide, but is richer in calcium oxide while the concentration of potassium oxide is at the same time significantly lower.

In particular, the implant according to the invention may contain magnesium or a magnesium alloy as the metal.

The present invention also relates to a process for preparing the coated implant according to the invention, comprising the following steps:
- providing an implant;
- converting the mixture of the coating material to a liquid or dissolved state;
- coating the implant with the mixture of the coating material, which is in a liquid state, by application in a spraying, depositing and/or immersion process;
- optionally compacting the applied layer by heat treatment; and
- optionally structuring the surface of the layer by subtractive and/or additive methods.

Liquid or dissolved state means that the mixture of the coating material is in a suspended state, i. e. the mixture of the coating material is in an aqueous medium. The aqueous medium may contain additionally at least one wetting agent, binding agent as suspension stabilizer for glazes or combinations thereof. Typically, the binding agent is a preparation of hydrocolloid which is water-miscible, has a density (20 °C) of approx. 1.1 g/cm³, and a viscosity (20 °C) of approx. 100 000 mPas as commercial available OPTAPIX G 1201.

A subtractive method for structuring the layer can be performed by etching the surface with chemicals which partially dissolve the surface.

An additive method is preferably performed by providing the coated implant with a second coating in particular with the same composition as the first one. The second coating is applied in a thinner thickness. The first coating is typically applied with a thickness of 30 to 100 µm, in particular about 50 - 80 µm and the second coating is typically applied with a thickness of 10 to 30 µm, in particular about 20 µm. The thickness is related with the coating before sintering. The sintering temperature for sintering the first coating is in the range of 790 - 880°C, in particular about 830 °C for 80 to 120 min, in particular about 100 min, and for the sintering of the second coating in the range of 740 - 780°C, in particular about 760°C for 5 to 20 min, in particular about 10 min. Performing this embodiment an implant with a microstructured surface can be manufactured.

The conversion of the mixture of the coating material to a liquid or dissolved state may be either the melting of the mixture by temperature treatment, or the dispersing of the mixture in a liquid for suspension, or the dissolving of the material in a suitable solvent. When the coatings are made of polymers, a liquid monomer constituting the coating can be applied to the implant surface and compacted on the implant by a polymerization reaction.

The present invention also relates to the use of the mixture according to the invention for preparing a coated implant according to the invention, especially a dental implant, which may be designed as a one-part or multi-part dental implant.
Figure 1 shows a typical implant.
Figure 2 shows the change of mass of a coating according to the invention on a substrate after storage in Simulated Body Fluid.
Figure 3 shows the change of mass of a coating according to the invention from storage in simulated saliva fluid with parallel abrasion by using the powder blasting device (simulation of conventional mechanical load from tooth cleaning).

The term "physiological conditions" means conditions that prevail, in particular, in the human oral cavity or in the human body in a non-pathological state. For example, saliva of body temperature is present in the oral cavity. Mechanical loads also occur, for example, from the chewing of food, but also during tooth cleaning.

The term "implant" means a component that is anchored in a jawbone by means of surgical measures and which serves either to receive a dental crown-like construction for replacing a missing tooth, or to anchor a removable prosthesis.

The term "coating" within the meaning of the invention means a deposit on the implant having a defined composition and a structured surface and being firmly bonded to the implant.

In the following, the term "enossal area" designates the area of the implant that is to be anchored in the jawbone. The term "ongrowth of the implant within the bone" within the meaning of the invention means that the bone grows closely to the surface of the enossal area of the implant on a microscopic scale, and thus a stable anchoring of the implant in the bone is achieved.

"Chemical removal" within the meaning of the invention means the removal of the microstructured surface of the implant with chemicals, saliva or liquids from food.

"Mechanical removal" is understood to mean abrading by instruments.

Within the meaning of the invention, "instruments in common use in medicine or dentistry" include those instruments that can be used in a dental practice for cleaning the surfaces of teeth or implants.

By "liquids occurring in the oral cavity", the skilled person understands saliva, which is naturally present in the oral cavity, but also a supplied liquid. In addition to the liquids usually supplied with the food, these may also be specific solutions, such as mouthwashes, weakly acidic solutions, solutions with chelating agents, etc.

A "ceramic" within the meaning of the invention is a predominantly crystalline inorganic, non-metallic material, for example, based on metal oxides, or nitrides of carbon or silicon.

Within the meaning of the invention, "glass" is to be understood as a predominantly non-crystalline inorganic, non-metallic material, for example, based on metal oxides, or nitrides of carbon or silicon.

### Example 1:

1. Preparing a glass by mixing the raw materials (e.g., silica glass powder, calcium hydrogenphosphate, calcium carbonate, magnesium oxide, potassium carbonate) in relative amounts for preparing a glass with the composition 45% by mass of SiO₂, 17% by mass of MgO, 22.5% by mass of CaO, 9.5% by mass of K₂O, 6% by mass of P₂O₅, homogeneously melting the raw materials in a platinum crucible at 1400 °C for 2 hours, quenching the molten glass in distilled water, and drying the resulting glass frit at 70 °C for 5 hours.
2. Grinding the dried glass frit in a ball mill with zirconia balls until the primary particle size of the glass is < 20 µm, measured using laser diffraction (ISO 13320).
3. Preparing an aqueous glass suspension with 49.375% by mass of glass powder, 49.375% by mass of distilled water, 1.0% by mass of organic liquefier (Optapix G1201, Zschimmer und Schwarz), 0.25% by mass of wetting agents (KG 9033, Zschimmer und Schwarz), dispersing the glass suspension by ultrasound and by stirring.
4. Spraying the aqueous glass suspension onto a zirconia implant by means of a spray gun until a layer of uniform thickness (ca. 50 µm, measured by investigating cross sections using Scanning Electron Microscopy) is formed. Subsequently drying.
5. Firing the implant coating in an oven under vacuum at 830 °C for 100 min.

### Example 2:

1. Preparing a glass by mixing the raw materials (e.g., silica glass powder, calcium hydrogenphosphate, calcium carbonate, magnesium oxide, potassium carbonate) in relative amounts for preparing a glass with the composition 45% by mass of SiO₂, 17% by mass of MgO, 22.5% by mass of CaO, 9.5% by mass of K₂O, 6% by mass of P₂O₅, homogeneously melting the raw materials in a platinum crucible at 1400 °C for 2 hours, quenching the molten glass in distilled water, and drying the resulting glass frit at 70 °C for 5 hours.
2. Grinding the dried glass frit in a ball mill with zirconia balls until the primary particle size of the glass is < 20 µm, measured using laser diffraction (ISO 13320).
3. Preparing an aqueous glass suspension with 49.375% by mass of glass powder, 49.375% by mass of distilled water, 1.0% by mass of organic liquefier (Optapix G1201, Zschimmer und Schwarz), 0.25% by mass of wetting agents (KG 9033, Zschimmer und Schwarz), dispersing the glass suspension by ultrasound and by stirring.
4. Immersing a zirconia implant into the aqueous glass suspension to deposit a uniform layer (ca. 50 µm, measured by investigating cross sections using Scanning Electron Microscopy) by dip coating. Subsequently drying.
5. Firing the implant coating in an oven under vacuum at 830 °C for 100 min.

### Example 3

### Microstructuring

A specimen as obtained by Example 1 or 2 has been coated a second time with the glass suspension, prepared as described in Example 1, using the spraying method, as described in Example 1, to deposit a second uniform layer (ca. 20 µm, measured by investigating cross sections using Scanning Electron Microscopy) onto the first, densely sintered glass coating. After drying at room temperature, the implant was fired in an oven at 760 °C for 10 min. This treatment caused a microstructure with a roughness of Ra = 5.38 µm ± 0.7 µm (according to ISO 4287:1997, mean and standard deviation, measured using Laser Scanning Microscopy).

### Example 4:

### Degradation studies in Simulated Body Fluid

The glass composition was: SiO₂ = 45% by weight, MgO = 17% by weight, CaO = 22.5% by weight, K₂O = 9.5% by weight, P₂O₅ = 6% by weight. The cylindrical specimens had a diameter of 15 ± 0.5 mm and a height of 1.0 ± 0.5 mm. The surface of the specimens was polished with a 3 µm diamond suspension. Simulated Body Fluid having the following composition was prepared.

**Composition of Simulated Body Fluid (SBF)**

| Component | Concentration |
|---|---|
| NaCl | 7.996 g/l |
| NaHCO₃ | 0.350 g/l |
| KCl | 0.224 g/l |
| K₂HPO₄ x 3H₂O | 0.228 g/l |
| MgCl₂ x 6 H₂O | 0.305 g/l |
| 1.0 M HCl | 40.0 cm³/l |
| CaCl₂ | 0.278 g/l |
| Na₂SO₄ | 0.071 g/l |
| Tris ((CH₂OH)₃CNH₂) | 6.057 g/l |

At a temperature of 37 °C, the pH of the SBF solution was adjusted to 7.40 with 1.0 M HCI. Each specimen was stored at 37 °C in 40 ml of SBF solution. The number of specimens tested in each period was n = 3. The following 5 periods were tested: 1 d, 3.5 d, 7 d, 14 d, 28 d. The masses of the specimens were determined before and after the storage, and the change of mass was calculated.

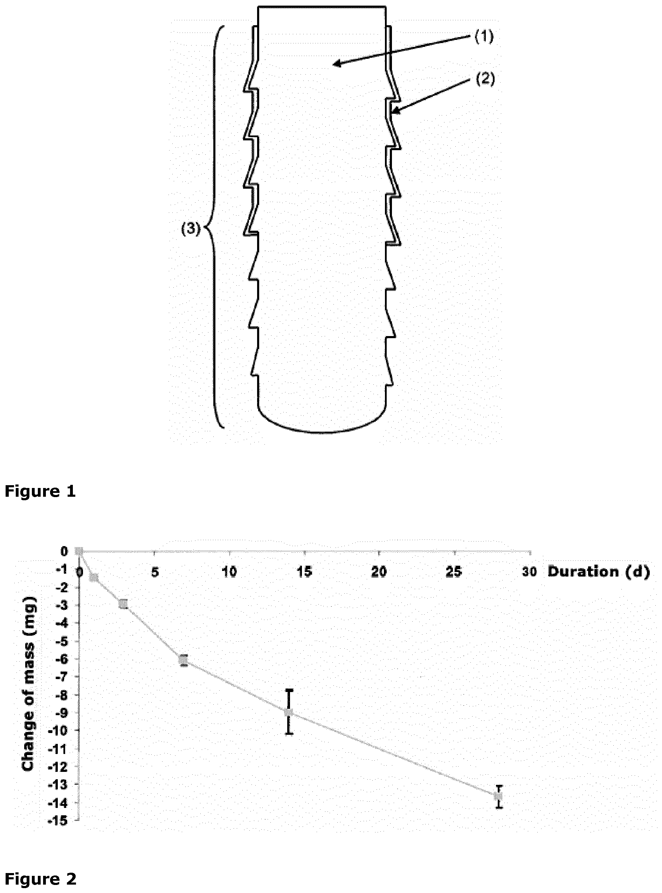

In SBF, a degradation of about 0.12 mg/mm² per day took place. Figure 2 shows the change of mass of the specimens after storage in Simulated Body Fluid.

### Example 5:

### Degradation studies in simulated saliva fluid

The glass composition was: SiO₂ = 45% by weight, MgO = 17% by weight, CaO = 22.5% by weight, K₂O = 9.5% by weight, P₂O₅ = 6% by weight. The cylindrical specimens had a diameter of 15 ± 0.5 mm and a height of 1.0 ± 0.5 mm. The surface of the specimens was polished with a 3 µm diamond suspension. A simulated saliva fluid (salive artificielle Gal-Fovet; SAGF) having the following composition was used:

**Composition of the simulated saliva fluid (SAGF)**

| Component | Concentration |
|---|---|
| NaCl | 125.6 mg/l |
| KCl | 963.9 mg/l |
| KSCN | 189.2 mg/l |
| KH₂PO₄ | 654.5 mg/l |
| Urea | 200.0 mg/l |
| Na₂SO₄ x 10H₂O | 763.2 mg/l |
| NH₄Cl | 178.0 mg/l |
| CaCl₂ x 2H₂O | 227.8 mg/l |
| NaHCO₃ | 630.8 mg/l |

At a temperature of 37 °C, the pH of the SAGF solution was adjusted to 7.40 with CO₂. Each specimen was stored at 37 °C in 50 ml of SAGF solution. After 3.5 days, the medium was changed, and the specimen surfaces were cleaned by means of a commercially available powder blasting device (PROPHYflex 2 2012, KaVo, Biberach an der Riss, Germany) to remove the forming layer. The number of specimens tested in each period was n = 5. The following 5 periods were tested: 1 d, 3.5 d, 7 d, 14 d, 28 d. The masses of the specimens were determined before and after the storage, and the change of mass was calculated.

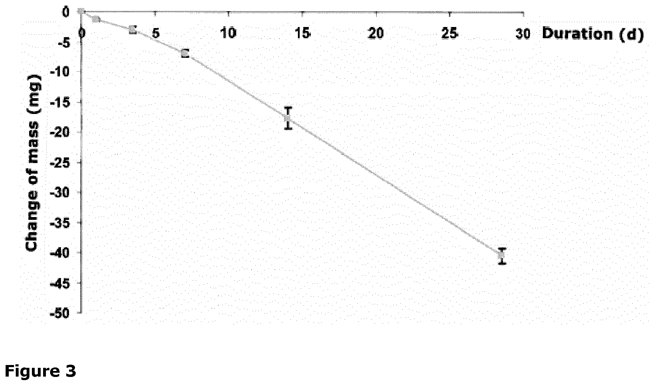

This treatment caused an abrasion of about 0.35 mg/mm² per day. Figure 3 shows the change of mass of the specimens from the storage in simulated saliva fluid with parallel abrasion from using the powder blasting device.

### References

1: Mombelli A, Lang NP. The diagnosis and treatment of peri-implantitis. Periodontol 2000. 1998;17:63-76.
2: Hohmann A, Hielscher. Lehrbuch der Zahntechnik. Quintessenz Bibliothek. 2004;2.
3: Le Guehennec L, Goyenvalle E, Lopez-Heredia MA, Weiss P, Amouriq Y, Layrolle P. Histomorphometric analysis of the osseointegration of four different implant surfaces in the fernoral epiphyses of rabbits. Clinical Oral Implants Research. 2008;19:1103.
4: Buser D, Schenk RK, Steinemann S, Fiorellini JP, Fox CH, Stich H. Influence of surface characteristics an bone integration of titanium implants. A histomorphometric study in miniature pigs. Journal of Biomedical Materials Research. 1991;25:889-902.
5: Shi G, Ren L, Wang L, Lin H, Wang S, Tong Y. H202/HCI and heat-treated Ti-6AI-4V stimulates pre-osteoblast proliferation and differentiation. Oral Surgery, Oral Medicine, Oral Pathology, Oral Radiology, and Endodontology. 2009; 108: 368-75.
6: Oh TJ, Yoon J, Misch CE, Wang HL. The causes of early implant bone loss: myth or science? Journal of periodontology. 2002;73:322-33.
7: Berglundh T, Gotfredsen K, Zitzmann NU, Lang NP, Lindhe J. Spontaneous progression of ligature induced peri-implantitis at implants with different surface roughness: an experimental study in dogs. Clinical Oral Implants Research. 2007;18:655-61.
8: Schwarz F, Bieling K, Bonsmann M, Latz T, Becker J. Nonsurgical treatment of moderate and advanced periimplantitis lesions: a controlled clinical study. Clinical Oral Investigations. 2006 Sep 13;10:279-88.
9: Mombelli A, Lang NP. Antimicrobial treatment of peri-implant infections. Clinical Oral Implants Research. 2002;3:162-8.
10: Claffey N, Clarke E, Polyzois I, Renvert S. Surgical treatment of peri-implantitis. Journal of clinical periodontology. 2008;35:316-32.

## Claims

1. An implant (1) comprising an enossal area (3) and provided with an inorganic coating (2), wherein the coating (2) is selected from the group consisting of ceramic, glass ceramic, glass which at least partially covers the enossal area (3), and the coating (2) facilitates the ongrowth of the implant (1) within the bone, **characterized in that** said inorganic coating can be removed chemically under physiological conditions by contact with liquids occurring in the oral cavity.

2. The implant according to claim 1, **characterized in that** said coating (1) can be removed by means of instruments in common use in medicine or dentistry.

3. The implant according to at least one of claims 1 or 2, **characterized in that** said implant is made of metal, ceramic or plastic or combinations thereof.

4. The implant according to at least one of claims 1-3, **characterized in that** said organic material is an organic polymer selected from the group consisting of polyesters (e.g., polylactide, polycaprolactone, poly(butylene succinate), poly(butylene terephthalate), poly(butylene adipate/butylene terephthalate), polyhydroxyalkanoate, poly(trimethylene terephthalate), aromatic-aliphatic copolyesters), vinyl polymers (e.g., poly(vinyl alcohol)), starch-based plastics (e.g., thermoplastic starch), and cellulose-based plastics (e.g., cellulose acetate, cellulose hydrate).

5. The implant according to claim 1, **characterized in that** said ceramic is a a calcium carbonate, a calcium silicate, or mixtures thereof.

6. The implant according to claim 1, **characterized in that** said glass ceramic is based on an alkali silicate glass, alkaline earth silicate glass, , or mixtures thereof, and is in a partially or completely crystallized form.

7. The implant according to claim 1, **characterized in that** said glass is an alkali silicate glass, alkaline earth silicate glass, , or mixtures thereof.

8. The implant according to claim 1, **characterized in that** said metal is magnesium or a magnesium alloy.

9. The implant according to at least one of claims land 5-8, **characterized in that** the coefficient of thermal expansion of the coating is ± 1 ppm/K, based on the coefficient of thermal expansion of the substrate material.

10. The implant according to at least one of the foregoing claims having a microstructured surface.

11. The implant according to at least one of the foregoing claims having a coating comprising or consisting of a composition of
SiO₂ in amounts of from 40 to 50% by mass;
MgO in amounts of from 25 to 30% by mass;
CaO in amounts of from 0 to 5% by mass;
K₂O in amounts of from 18 to 24% by mass;
P₂O₅ in amounts of from 0 to 10% by mass;
Na₂O in amounts of from 0 to 5% by mass;
ZrO₂ in amounts of from 0 to 5% by mass;
SrO in amounts of from 0 to 5% by mass;
BaO in amounts of from 0 to 5% by mass;
Al₂O₃ in amounts of from 0 to 3% by mass;
Y₂O₃ in amounts of from 0 to 5% by mass;
CaF₂ in amounts of from 0 to 5% by mass;
TiO₂ in amounts of from 0 to 5% by mass;
Ag in amounts of from 0 to 5% by mass
or
SiO₂ in amounts of from 40 to 50% by mass;
MgO in amounts of from 14 to 20% by mass;
CaO in amounts of from 20 to 25% by mass, in particular 20 to 23% by mass;
K₂O in amounts of from 7 to 12% by mass;
P₂O₅ in amounts of from 0 to 10% by mass, in particular 1 to 10% by mass;
Na₂O in amounts of from 0 to 5% by mass;
ZrO₂ in amounts of from 0 to 5% by mass;
SrO in amounts of from 0 to 5% by mass;
BaO in amounts of from 0 to 5% by mass;
Al₂O₃ in amounts of from 0 to 3% by mass;
Y₂O₃ in amounts of from 0 to 5% by mass;
CaF₂ in amounts of from 0 to 5% by mass;
TiO₂ in amounts of from 0 to 5% by mass;
Ag in amounts of from 0 to 5% by mass
or
SiO₂ in amounts of 43 to 46 % by mass, in particular 45 % by mass; MgO in amounts of 15 to 19 % by mass, in particular 17 % by mass; CaO in amounts of 21 to 24 % by mass, in particular 22.5 % by mass; K₂O in amounts of 8 to 11 % by mass, in particular 9.5 % by mass; P₂O₅ in amounts of 5 to 7 % by mass, in particular 6 % by mass.

12. A process for manufacturing a coated implant according to at least one of claims 1-11, comprising the following steps:
- providing an implant;
- converting the mixture of the coating material to a liquid or dissolved state;
- coating the implant with the mixture of the coating material, which is in a liquid state, by application in a spraying, depositing and/or immersion process;
- optionally compacting the applied layer by heat treatment; and
- optionally structuring the surface of the layer by subtractive and/or additive methods.

## Patentansprüche

1. Implantat (1) mit einem enossalen Bereich (3), das mit einer anorganischen Beschichtung (2) versehen ist, wobei die Beschichtung (2) aus der Gruppe ausgewählt ist, die aus Keramik, Glaskeramik, Glas besteht, und mindestens teilweise den enossalen Bereich (3) abdeckt und die Beschichtung (2) das Einwachsen des Implantats (1) im Knochen erleichtert, **dadurch gekennzeichnet, dass** die anorganische Beschichtung unter physiologischen Bedingungen durch Kontakt mit Flüssigkeiten, die in der Mundhöhle vorkommen, chemisch entfernbar ist.

2. Implantat gemäß Anspruch 1, **dadurch gekennzeichnet, dass** die Beschichtung (1) mittels in der Medizin oder Zahnmedizin gebräuchlichen Instrumenten entfernbar ist.

3. Implantat gemäß wenigstens einem der Ansprüche 1 oder 2, **dadurch gekennzeichnet, dass** das Implantat aus Metall, Keramik, oder Kunststoff oder Kombinationen davon besteht.

4. Implantat gemäß wenigstens einem der Ansprüche 1 bis 3, **dadurch gekennzeichnet, dass** das organische Material ein organisches Polymer ist, das aus der Gruppe ausgewählt ist, die aus Polyestern (z.B. Polylactid, Polycaprolacton, Polybutylensuccinat, Polybutylenterephthalat, Polybutylenadipat-butylenterephthalat, Polyhydroxyalkanoat, Polytrimethylenterephthalat, aromatisch-aliphatische Copolyester), Vinylpolymeren (z.B. Polyvinylalkohol), stärkebasierten Kunststoffen (z.B. thermoplastische Stärke) und cellulosebasierten Kunststoffen (z.B. Celluloseacetat, Cellulosehydrat) besteht.

5. Implantat gemäß Anspruch 1, **dadurch gekennzeichnet, dass** es sich bei der Keramik um ein Calciumcarbonat, ein Calciumsilicat oder Mischungen davon handelt.

6. Implantat gemäß Anspruch 1, **dadurch gekennzeichnet, dass** die Glaskeramik auf einem Alkali-Silicatglas, Erdalkali-Silicatglas oder Mischungen daraus basiert und teilweise oder vollständig kristallisiert vorliegt.

7. Implantat gemäß Anspruch 1, **dadurch gekennzeichnet, dass** es sich bei dem Glas um ein Alkali-Silicatglas, Erdalkali-Silicatglas oder Mischungen davon handelt.

8. Implantat gemäß Anspruch 1, **dadurch gekennzeichnet, dass** das Metall Magnesium oder eine Magnesiumlegierung ist.

9. Implantat gemäß wenigstens einem der Ansprüche 1 und 5 bis 8, **dadurch gekennzeichnet, dass** der Wärmeausdehnungskoeffizient der Beschichtung ± 1 ppm/K beträgt, bezogen auf den Wärmeausdehnungskoeffizienten des Substratmaterials.

10. Implantat gemäß wenigstens einem der vorstehenden Ansprüche, das eine mikrostrukturierte Oberfläche aufweist.

11. Implantat gemäß wenigstens einem der vorstehenden Ansprüche, das eine Beschichtung aufweist, die die folgende Zusammensetzung umfasst oder daraus besteht:
SiO₂ in Mengen von 40 bis 50 Massen-%;
MgO in Mengen von 25 bis 30 Massen-%;
CaO in Mengen von 0 bis 5 Massen-%;
K₂O in Mengen von 18 bis 24 Massen-%;
P₂O₅ Mengen von 0 bis 10 Massen-%;
Na₂O in Mengen von 0 bis 5 Massen-%;
ZrO₂ in Mengen von 0 bis 5 Massen-%;
SrO in Mengen von 0 bis 5 Massen-%;
BaO in Mengen von 0 bis 5 Massen-%;
Al₂O₃ in Mengen von 0 bis 3 Massen-%;
Y₂O₃ in Mengen von 0 bis 5 Massen-%;
CaF₂ in Mengen von 0 bis 5 Massen-%;
TiO₂ in Mengen von 0 bis 5 Massen-%;
Ag in Mengen von 0 bis 5 Massen-%;
oder
SiO₂ in Mengen von 40 bis 50 Massen-%;
MgO in Mengen von 14 bis 20 Massen-%;
CaO in Mengen von 20 bis 25 Massen-%, insbesondere 20 bis 23 Massen-%;
K₂O in Mengen von 7 bis 12 Massen-%;
P₂O₅ Mengen von 0 bis 10 Massen-%, insbesondere 1 bis 10 Massen-%;
Na₂O in Mengen von 0 bis 5 Massen-%;
ZrO₂ in Mengen von 0 bis 5 Massen-%;
SrO in Mengen von 0 bis 5 Massen-%;
BaO in Mengen von 0 bis 5 Massen-%;
Al₂O₃ in Mengen von 0 bis 3 Massen-%;
Y₂O₃ in Mengen von 0 bis 5 Massen-%;
CaF₂ in Mengen von 0 bis 5 Massen-%;
TiO₂ in Mengen von 0 bis 5 Massen-%;
Ag in Mengen von 0 bis 5 Massen-%;
oder
SiO₂ in Mengen von 43 bis 46 Massen-%, insbesondere 45 Massen-%; MgO in Mengen von 15 bis 19 Massen-%, insbesondere 17 Massen-%; CaO in Mengen von 21 bis 24 Massen-%, insbesondere 22,5 Massen-%; K₂O in Mengen von 8 bis 11 Massen-%, insbesondere 9,5 Massen-%; P₂O₅ in Mengen von 5 bis 7 Massen-%, insbesondere 6 Massen-%.

12. Verfahren zur Herstellung eines beschichteten Implantats gemäß mindestens einem der Ansprüche 1-11 mit den folgenden Schritten:
- Bereitstellen eines Implantats;
- Überführen der Mischung des Beschichtungsmaterials in einen flüssigen oder gelösten Zustand;
- Beschichten des Implantats mit der Mischung des Beschichtungsmaterials im flüssigen Zustand durch Auftragen in einem Sprüh-, Abscheide- und/oder Tauchvorgang;
- gegebenenfalls Verdichtung der aufgetragenen Schicht durch Wärmebehandlung; und
- gegebenenfalls Oberflächenstrukturierung der Schicht durch subtraktive und/oder additive Verfahren.

## Revendications

1. Implant (1) comprenant une région endo-osseuse (3) et portant un revêtement inorganique (2), dans lequel le revêtement (2) est choisi dans le groupe consistant en céramique, vitrocéramique, verre, couvrant la région endo-osseuse (3) au moins partiellement, et le revêtement (2) facilite l'adhérence de l'implant (1) au sein de l'os, **caractérisé en ce que** ledit revêtement inorganique peut être éliminé chimiquement sous conditions physiologiques par contact avec des liquides se produisant dans la cavité orale.

2. Implant selon la revendication 1, **caractérisé en ce que** ledit revêtement (1) peut être éliminé en utilisant des instruments couramment utilisés en médecine et en dentisterie.

3. Implant selon l'une au moins des revendications 1 ou 2, **caractérisé en ce que** ledit implant est en métal, en céramique ou en plastique, ou en des combinaisons de ceux-ci.

4. Implant selon l'une au moins des revendications 1 à 3, **caractérisé en ce que** ledit matériau organique est un polymère organique choisi dans le groupe consistant en polyesters (p. ex., polylactide, polycaprolactone, poly(succinate de butylène), poly(téréphtalate de butylène), poly(adipate de butylène/téréphtalate de butylène), polyhydroxyalkanoate, poly(téréphtalate de triméthylène), copolyesters aromatique-aliphatique), polymères de vinyle (p. ex., alcool polyvinylique), plastiques à base d'amidon (p. ex., amidon thermoplastique), et plastiques à base de cellulose (p. ex., acétate de cellulose, hydrate de cellulose).

5. Implant selon la revendication 1, **caractérisé en ce que** ladite céramique est un carbonate de calcium, un silicate de calcium, ou des mélanges de ceux-ci.

6. Implant selon la revendication 1, **caractérisé en ce que** ladite vitrocéramique est basée sur un verre de silicate alcalin, un verre de silicate alcalino-terreux, ou des mélanges de ceux-ci, et qu'elle est sous forme cristallisée partiellement ou complètement.

7. Implant selon la revendication 1, **caractérisé en ce que** ledit verre est un verre de silicate alcalin, un verre de silicate alcalino-terreux, ou des mélanges de ceux-ci.

8. Implant selon la revendication 1, **caractérisé en ce que** ledit métal est le magnésium ou un alliage de magnésium.

9. Implant selon l'une au moins des revendications 1 et 5 à 8, **caractérisé en ce que** le coefficient de dilatation thermique du revêtement est ± 1 ppm/K, par rapport au coefficient de dilatation thermique du matériau de substrat.

10. Implant selon l'une au moins des revendications précédentes ayant une surface microstructurée.

11. Implant selon l'une au moins des revendications précédentes ayant un revêtement comprenant ou consistant en une composition de
SiO₂ en des quantités de 40 à 50 % en masse,
MgO en des quantités de 25 à 30 % en masse,
CaO en des quantités de 0 à 5 % en masse,
K₂O en des quantités de 18 à 24 % en masse,
P₂O₅ en des quantités de 0 à 10 % en masse,
Na₂O en des quantités de 0 à 5 % en masse,
ZrO₂ en des quantités de 0 à 5 % en masse,
SrO en des quantités de 0 à 5 % en masse,
BaO en des quantités de 0 à 5 % en masse,
Al₂O₃ en des quantités de 0 à 3 % en masse,
Y₂O₃ en des quantités de 0 à 5 % en masse,
CaF₂ en des quantités de 0 à 5 % en masse,
TiO₂ en des quantités de 0 à 5 % en masse,
Ag en des quantités de 0 à 5 % en masse,
ou
SiO₂ en des quantités de 40 à 50 % en masse,
MgO en des quantités de 14 à 20 % en masse,
CaO en des quantités de 20 à 25 % en masse, notamment 20 à 23 % en masse,
K₂O en des quantités de 7 à 12 % en masse,
P₂O₅ en des quantités de 0 à 10 % en masse, notamment 1 à 10 % en masse,
Na₂O en des quantités de 0 à 5 % en masse,
ZrO₂ en des quantités de 0 à 5 % en masse,
SrO en des quantités de 0 à 5 % en masse,
BaO en des quantités de 0 à 5 % en masse,
Al₂O₃ en des quantités de 0 à 3 % en masse,
Y₂O₃ en des quantités de 0 à 5 % en masse,
CaF₂ en des quantités de 0 à 5 % en masse,
TiO₂ en des quantités de 0 à 5 % en masse,
Ag en des quantités de 0 à 5 % en masse,
ou
SiO₂ en des quantités de 43 à 46 % en masse, notamment 45 % en masse, MgO en des quantités de 15 à 19 % en masse, notamment 17 % en masse, CaO en des quantités de 21 à 24 % en masse, notamment 22,5 % en masse, K₂O en des quantités de 8 à 11 % en masse, notamment 9,5 % en masse, P₂O₅ en des quantités de 5 à 7 % en masse, notamment 6 % en masse.

12. Procédé pour fabriquer un implant revêtu selon l'une au moins des revendications 1 à 11, comprenant les étapes suivantes consistant à :
- procurer un implant,
- convertir le mélange du matériau de revêtement en un état liquide ou dissout,
- enrober l'implant avec le mélange du matériau de revêtement, qui est en état liquide, par application dans un procédé de pulvérisation, de dépôt, et/ou d'immersion,
- éventuellement compacter la couche appliquée par traitement thermique, et
- éventuellement structurer la surface de la couche par des procédés soustractifs et/ou additifs.
